(19)

Europäisches
Patentamt
European
Patent Office
Office européen
des brevets

(11)  **EP 2 982 975 A1**

(12)  **EUROPEAN PATENT APPLICATION**

(43) Date of publication:
**10.02.2016 Bulletin 2016/06**

(51) Int Cl.:
**G01N 33/00** $^{(2006.01)}$

(21) Application number: **14306246.1**

(22) Date of filing: **06.08.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicants:
• **INSTITUT PASTEUR**
**75015 Paris (FR)**
• **INSTITUT NATIONAL DE LA RECHERCHE
AGRONOMIQUE**
**75007 Paris (FR)**

(72) Inventors:
• **Masson, Jean-Baptiste**
**75015 PARIS (FR)**
• **Lucas, Philippe**
**78960 VOISINS LE BRETONNEUX (FR)**
• **Touche, Alexandre**
**75017 PARIS (FR)**

(74) Representative: **Santarelli**
**49, avenue des Champs-Elysées**
**75008 Paris (FR)**

(54)  **Method and device for monitoring detection of odors by moving animals or by moving apparatus in turbulent flows**

(57)    The present invention provides a device that can be transported by a moving animal or by a moving apparatus, such as a robot, for real-time monitoring of odor detection in turbulent flows. The device comprises connecting means (200) for connecting a portion of biological material, such as an insect antenna, that is sensitive to a predetermined molecule, such as a pheromone, to the device, the portion of biological material producing a specific electrical signal, like an electroantennogram signal, in the presence of the at least one predetermined molecule. The device also comprises amplification means (205, 220) for amplifying an electrical signal received from the connecting means and providing an amplified signal to computation means. The device further comprises computation means (250) for filtering an amplified signal received from the amplification means, the filtering comprising a step of applying a temporal high-pass filter to the amplified signal received from the amplification means. Insect antennae attached to an autonomous robot and used for electroantennographic detection. An insect antenna mounted on a robot is used for olfactive searches using a mapless search scheme in order to reproduce olfactive search strategies used by insects which have a limited space perception.

Fig. 6

- 600 — Determining a mapless search function $F_t(\Theta_t)$
- 605 — Obtaining measurement of propagation at sensor location ($\vec{r}_t$)
- 610 — Source located? — yes → end / no
- 615 — Determining a set of $\vec{r}^{\,i}_{t+dt}$
- 620 — Determining $\vec{r}_{t+dt}$ minimizing $F_t(\vec{r}_t \rightarrow \vec{r}_{t+dt} |?_t)$ for each $\vec{r}^{\,i}_{t+dt}$ of the set
- 625 — Moving sensor to $\vec{r}_{t+dt}$
- 610' — Source located? — yes → end / no

EP 2 982 975 A1

**Description**

FIELD OF THE INVENTION

The present invention relates to the detection of odor. Particularly, but not exclusively, the invention relates to a method and a device for monitoring detection of odors by moving animals or by moving apparatus in turbulent flows. Such a method and a device can be combined with system for locating an emitting source of which measurements of emission propagation at locations different from that of the emitting source can be obtained from those locations, lacking space perception.

BACKGROUND OF THE INVENTION

Insects and various animals are able to track sources of food or pheromone over large distances by crawling, walking and/or flying. Even though turbulence prevents the formation of stable odor and/or pheromone gradients, animals are able to locate sources of odor or pheromone by detecting discontinuous patches of odor or pheromone whose local gradients do not point towards the sources, that is to say to locate sources of odor while spending large amount of time without detecting the odor that sources are searched for.

Furthermore, it is to be noted that insect space perception is very limited. As a consequence, one wonders how to carry out efficient searches without space processing capacities. Basic search strategies can be based on "zigzagging/casting" behavior according to which it is assumed that when not any odor detection is made, a lateral path with respect to the wind direction is chosen so as to increase the odor detection probability and when an odor detection is made, an upwind path is selected to reach the source. However, such strategies appear to be efficient only for short range searches (e.g. a few meters).

Accordingly, in order to better understand olfactory search strategies of animals and thus, to optimize olfactory searching strategies of devices such as robots, there is a need to determine the odor path an insect experienced to link its behavior to the odor path processing.

Moreover, knowing searching strategies of insects may lead to improving numerous applications among which identifying better search strategies for locating chemical leaks, improving flight strategy of small micro-air-vehicle, and using insects to search specific compounds. It is also to be noted that combining searching strategies of different insects, for example walking and flying insects, may allow semi-active searches in complex environments.

Most experiments investigating olfaction and flying motion are performed in tethering assays. An animal is mechanically tethered and a virtual environment is designed to match as well as possible the natural environment of the animal. Olfactory stimuli are delivered through controllable valves that allow various odorized and non-odorized flows to reach the animal. Finally, various systems are added to ensure fast removal of odor to avoid contamination of the setups, especially for pheromone stimuli, generally without any guarantee that there is no contamination of the surrounding environment.

Measuring odor stimuli in the setups can be achieved with Photo Ionizing Detectors (PID) that measures volatile organic compounds and other gases in concentrations from sub parts per billion to 10,000 parts per million and produces instantaneous readings. According to this technology, molecules are ionized, typically by using UV light, leading to positively charged molecules, and then, a current is measured and associated with a concentration of molecules in the air. There exist PIDs that can operate up to a frequency of 300Hz. Detection concentration limits depend on the nature of the compounds that are measured. The PIDs typically weight between 0.8 and 1.0kg and require 110 or 220V power supply to operate, allowing free moving experiments only for large animals. The PIDs are mostly used in tethering experiments.

It is to be noted that PIDs are limited by the ionization energy of the molecules of interest (that should be typically less than 10.6 eV). Accordingly, most of the pheromones and of the volatile molecules (odors) cannot be detected or only for concentrations above the ones generating normal response and behavior of the animals. Therefore, it is worth noting that in the case of *Drosophila* animal that is frequently used for experiments, a lot of odors inducing a measurable behavior cannot be detected by PIDs.

Another experimental limitation is that PIDs cannot operate for long times with humidified air that is necessary to prevent either anomalies in animal behavior or drying out of insects' antenna.

In the document entitled *"Odour-plume dynamics influence the brain's olfactory code",* dated March 22, 2001, the authors, Vickers et al., present the orientation dynamics of moths in a 1-meter wind tunnel. In this document, the role of the turbulence is pointed out and the plume dynamic is described to be one of the most important elements in the olfactory search scheme. The authors use *H. virescens* antenna to record the plume dynamic statically at various positions in the wind tunnel. Furthermore, they use a third antenna glued to the head of the insect to record the odor detections encountered by the moth during the fight. This antenna is connected to an amplifier located outside a Faraday cage isolating the moth and the antenna from ambient electric perturbation so as to enable recording EAG signal for few dozen of centimeters.

The present invention has been devised to address one or more of the foregoing concerns.

SUMMARY OF THE INVENTION

According to a first aspect of the invention there is provided a mobile device for real-time monitoring of odor detection by a moving animal or by a moving apparatus, in turbulent flows, the device comprising:

- connecting means for connecting to the device at least a portion of biological material that is sensitive to at least one predetermined molecule, the at least a portion of biological material producing a specific electrical signal in the presence of the at least one predetermined molecule;
- amplification means for amplifying an electrical signal received from the connecting means and providing an amplified signal to computation means; and
- computation means for filtering an amplified signal received from the amplification means, the filtering comprising a step of applying a temporal high-pass filter to the amplified signal received from the amplification means.

The device of the invention makes it possible to measure odor detection of moving animals or moving apparatus in turbulent flows. It can be used, in particular, for studying the behavior of animals or for locating source of particles, molecules, or fragments of molecules.

In an embodiment, the computation means are further configured for generating a signal representing the detection or absence of detection of the at least one predetermined molecule, as a function of the filtered amplified signal.

In an embodiment, the amplification means comprise at least one of an instrument amplifier and an operational amplifier.

In an embodiment, the device further comprises wireless emitting means for emitting a signal obtained in the computation means as a function of the amplified signal received from the amplification means.

In an embodiment, the device further comprises powering means, the powering means comprising a power circuit for providing a virtual ground.

In an embodiment, the electrical signal received from the connecting means is of the ElectroAntennoGram type.

In an embodiment, the device further comprises a circuitry for modelling the at least a portion of biological material as a source of voltage.

In an embodiment, the device further comprises the at least a portion of biological material, the at least a portion of biological material including at least a portion of an insect antenna.

In an embodiment, the connecting means are configured for connecting a plurality of portions of biological material to the device, each of the portions of biological material being sensitive to at least one predetermined molecule and producing a specific electrical signal in the presence of the predetermined molecule, each of the portions of biological material providing a specific temporal dynamic and a specific sensibility in the presence of the predetermined molecule to which the portion of biological material is sensitive.

In an embodiment, the device further comprises actuator controller means for controlling displacements of an apparatus comprising the device, the actuator controller means providing a control signal that is at least partially determined as a function of the signal representing the detection or absence of detection of the at least one predetermined molecule.

In an embodiment, the computation means are further configured for:

- computing, for a plurality of possible next locations of the device, a free energy variation for moving the device from its current location to each of the plurality of possible next locations, the free energy being computed as a function of a standardized projected probability field of the location of a diffusing source of the at least one predetermined molecule, based on previous emission propagation measurements of the at least one predetermined molecule, the previous emission propagation measurements being obtained along a self-generated path of the device,
- determining a minimum free energy variation value amongst the computed free energy variations; and
- identifying the location associated with the determined minimum free energy variation as being the next location of the device.

A second aspect of the invention provides a method to be carried out in a mobile device for real-time monitoring of odor detection by a moving animal or by a moving apparatus, in turbulent flows, the method comprising the steps of:

- receiving an electrical signal from at least a portion of biological material that is sensitive to at least one predetermined molecule, the at least a portion of biological material producing a specific electrical signal in the presence of the at least one predetermined molecule;

- amplifying the received electrical signal; and
- filtering the amplified signal, the filtering step comprising a step of applying a temporal high-pass filter to the amplified received signal.

The method of the invention makes it possible to measure odor detection of moving animals or moving apparatus in turbulent flows. It can be used, in particular, for studying the behavior of animals or for locating source of particles, molecules, or fragments of molecules.

In an embodiment, the method further comprises a step of wirelessly emitting the filtered signal.

In an embodiment, the method further comprises a step of generating a signal representing the detection or absence of detection of the at least one predetermined molecule as a function of the filtered amplified signal.

In an embodiment, the method further comprises a step of wirelessly emitting the signal representing the detection or absence of detection of the at least one predetermined molecule.

In an embodiment, the method further comprises a step of controlling a displacement actuator at least partially as a function of the signal representing the detection or absence of detection of the at least one predetermined molecule.

In an embodiment, the method further comprises the steps of:

- computing, for a plurality of possible next locations of the device, a free energy variation for moving the device from its current location to each of the plurality of possible next locations, the free energy being computed as a function of a standardized projected probability field of the location of a diffusing source of the at least one predetermined molecule, based on previous emission propagation measurements of the at least one predetermined molecule,
- determining a minimum free energy variation value amongst the computed free energy variations; and
- identifying the location associated with the determined minimum free energy variation as being the next location of the device.

In an embodiment, the electrical signal received from the at least a portion of biological material is of the ElectroAntennoGram type.

In an embodiment, the at least one predetermined molecule is mixed with particles, molecules, or fragments of molecules that a source is to be located.

At least parts of the methods according to the invention may be computer implemented. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system". Furthermore, the present invention may take the form of a computer program product embodied in any tangible medium of expression having computer usable program code embodied in the medium.

Since the present invention can be implemented in software, the present invention can be embodied as computer readable code for provision to a programmable apparatus on any suitable carrier medium. A tangible carrier medium may comprise a storage medium such as a floppy disk, a CD-ROM, a hard disk drive, a magnetic tape device or a solid state memory device and the like. A transient carrier medium may include a signal such as an electrical signal, an electronic signal, an optical signal, an acoustic signal, a magnetic signal or an electromagnetic signal, e.g. a microwave or RF signal.

BRIEF DESCRIPTION OF THE DRAWINGS

Embodiments of the invention will now be described, by way of example only, and with reference to the following drawings in which:

Figure 1, comprising Figures 1a and 1b, illustrates an insect and a drone, respectively, to which is attached an electronic board connected to a cut antenna of an insect that is used as a chemical sensor for detecting odors;

Figure 2 schematically illustrates an example of a printed circuit board for amplifying a signal issued from an insect antenna;

Figure 3, comprising Figures 3a, 3b, and 3C, illustrates the transfer function, the structural noise, and the pulse responses of the printed circuit board illustrated in Figure 2, respectively;

Figure 4, comprising Figures 4a to 4d, illustrates results obtained by carrying out experiments with a hissing cockroach provided with a printed circuit connected board to which is connected a *Agrotis ipsilon* cut antenna;

Figure 5 illustrates an example of the architecture of a searching system according to a particular embodiment;

Figure 6 illustrates steps of an algorithm to search for an emitting source of which measurements of emission propagation at locations different from that of the emitting source can be obtained from those locations, in a highly variable environment, lacking space perception, according to an embodiment; and

**Figure 7,** comprising Figures 7a and 7b, illustrates an advantage of using several robots to locate one or several emitting sources. Figure 7a illustrates the variation of the average searching time as a function of the average distance between the robots and the centers of two emitting sources and as a function of the number of robots used. Figure 7b illustrates the variation of the average searching time as a function of the number of robots and as a function of the distance between the robots and the centers of two emitting sources.

## DETAILED DESCRIPTION OF THE INVENTION

According to a particular embodiment of the invention, simple and efficient method and device to monitor in real-time detection of odors (i.e. olfactory molecules) by a moving animal or by a moving apparatus in turbulent flows are provided. The method and the device are based on the use of insect's antenna or of another biological material allowing odor detection (e.g. the olfactory epithelium) in an open environment, as a chemical sensor coupled to an electronic board, typically mostly analogical, allowing the amplification of the signal issued by the sensor.

The insect's antenna and the biological material to be used are sensitive to at least one predetermined molecule, typically a predetermined pheromone, the insect's antenna and the biological material producing a specific electrical signal in the presence of these predetermined molecules. Pheromone ensures high selectivity of the signal generated by the antenna.

For the sake of illustration, an ElectroAntennoGram (EAG) signal response of an animal cut antenna can be used as an input signal to the electronic amplifier board. It has been observed that in experimental conditions, the EAG signal of the *Agrotis ipsilon* moth typically varies from 100 to 1,000 $\mu$V (which is an analog signal easily measurable). It has also been observed that a detectable EAG signal can be generated for several decades of pheromone concentration.

Furthermore, exploiting a property of passive scalar turbulence by mixing the compound detected by the animal with a molecule detected by the antenna used as a chemical sensor, especially a pheromone, ensures that the detection of a molecule plume by the antenna, especially a pheromone plume, corresponds to the detection of an odor plume by the animal.

Still exploiting this property, a molecule, especially a pheromone, detected by the antenna used as a chemical sensor can be mixed with particles, molecules, or fragments of molecules that source is to be located since the detection of the pheromone can be exploited by a mobile robot or drone to locate the source.

It is to be recalled that the electroantennography is a technique for measuring the average output graded field potential of an insect antenna to its brain for a given odor. It is generally performed by removing an antenna from the animal and connecting two electrodes to the ends of the antenna to measure voltage. Alternately, it can be performed by grounding the animal body and placing an electrode to the tip of the antenna for measuring voltage.

It is also to be recalled that insects detect odors with olfactory receptor neurons (ORNs) located within sensilla. ORNs can be extremely sensitive. For example, a single pheromone molecule was found to be sufficient to elicit an action potential in moth ORNs. The EAG signal can be measured from an insect antenna (in response to olfactory stimuli) using a pair of glass electrodes, the position of which being determined as a function of the shape of the insect antenna (e.g.flagellum (*lepidoptera*), lamellates *(scarab beetles),* or globular (*diptera*)).

For the sake of illustration, an EAG signal can be obtained by connecting one of the two electrodes, referred to as the recording electrode, to the cut tip of the antenna for insects having flagellate antennae (e.g. moths) or by inserting it within the antennal sensory surface (e.g. weevils). The other of the two electrodes, referred to as the reference electrode, is preferably inserted in the insect body (e.g. neck or eye) or it can be connected to the base of the antenna when isolated. The EAG signal can be understood as the incoherent sum of ORN depolarization states.

Regarding the *Agrotis ipsilon* (i.e. a moth), the time evolution of an EAG signal matches the one of the pheromone puffs.

EAG signals measured inside a Faraday cage typically vary from 0 to a few mV in response to several decades of pheromone concentration. Without electric protection and by connecting electrodes to the insect antenna and to a local electronic board amplifying the signal, attached to the insect, an EAG signal recorded on the board typically vary from 0.1 to 1 mV.

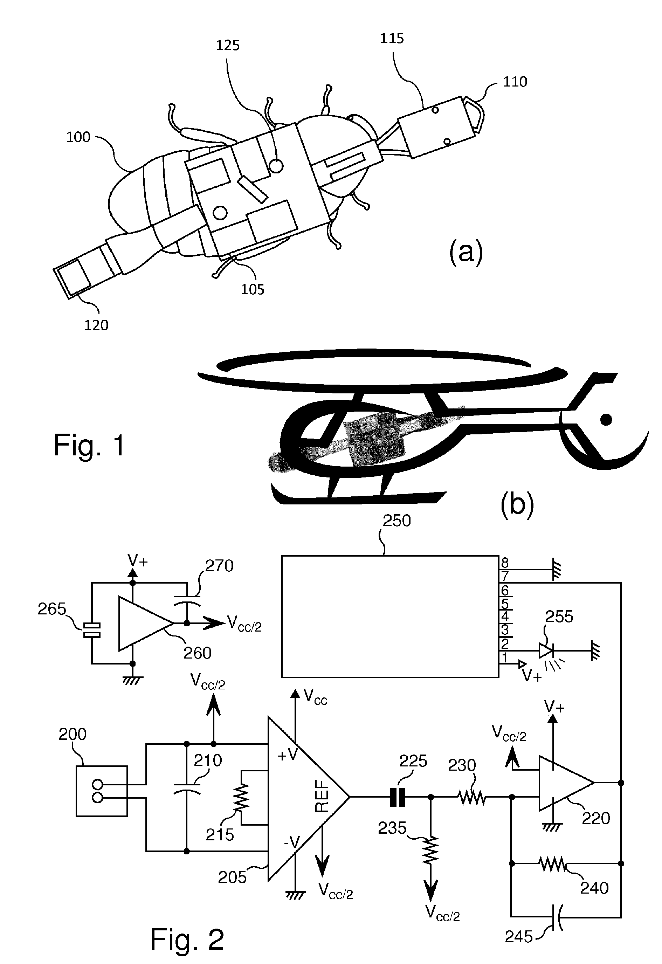

**Figure 1,** comprising Figures 1a and 1b, illustrates an insect and a drone, respectively, to which is attached an electronic amplifying board connected to a cut antenna of an insect that is used as a chemical sensor for detecting predetermined odors.

For the sake of illustration, the insect represented in Figure 1a is a cockroach 100 provided with a printed circuit board 105 that is connected to cut antenna 110 via connector 115. Printed board circuit 105 is powered with battery 120.

Still for the sake of illustration, printed board circuit 105 is provided with a led 125 that illuminates when an odor is detected. An example of printed board circuit 105 is described by reference to Figure 2.

By using a cut antenna of the same insect as insect 100, it is possible to correlate the detection of odor that is carried out by printed board circuit 105 with the insect behavior. Alternately, by mixing an odor exciting the cut antenna with an odor exciting the insect to which is fasten the cut antenna and the attached electronic, one can correlate the detection of odor that is carried out by printed board circuit 105 with the insect behavior.

Then, by imitating the behavior of an animal such as the insect illustrated in Figure 1 in view of odor detection, or by using a system for locating an emitting source of which measurements of emission propagation at locations different from that of the emitting source can be obtained from those locations, lacking space perception such as the one disclosed in published Patent Application US 2009/271125, one can control a moving apparatus such as a robot or a drone as a function of detecting an odor.

An example of such a drone equipped with a printed board circuit similar to printed board circuit 105 illustrated in Figure 1a, connected to a cut antenna or another biological material that can detect odors, is illustrated in Figure 1 b.

As mentioned previously, by mixing an odor that can be detected by the chemical sensor (i.e. a cut antenna or another biological material) associated with the used printed circuit board circuit, typically a pheromone, to particles, molecules or fragments of molecules that sources should be located, for example particles, molecules or fragments of molecules that can only be detected with difficulty, one can drive an apparatus such as a drone as a function of the detection of the odor to locate the sources.

It is to be noted that the odor detection process to be carried out by the printed circuit board 105 should comply with several challenging conditions among which the followings:

- the characteristic time of the odor plume dynamics that is about 0.1 to 10s (which is very slow from the electronic point of view),
- the impossibility of blocking static and non-static electric fields (there is no Faraday cage), and
- the strong capacitive coupling throughout the board.

By considering elements of passive scalar turbulence related to the capacity of plume detections by the chemical sensor associated with the electronic board (also called printed circuit board or PCB) and by defining the distance from the sensor to the source with variable x, the direction perpendicular to the one from the sensor to the source with axis y, the average velocity of the wind with variable $v$, the characteristic fluctuation of wind velocity with variable $\delta v$, and the characteristic size of the source with parameter a, and assuming that the wind blows in the x direction, one can determine the scaling factors characterizing time and concentration evolution. If the concentration of the source in the air is equal to $c_0$, the average concentration scales can be defined with the following relation:

$$\frac{c}{c_0} \sim \left(\frac{v}{\delta v}\right)^2 \cdot \left(\frac{a}{x}\right)^2$$

and the duration $\Delta t$ of the plume is such that:

$$\Delta t_{min} = \left(\frac{a^2 v}{\delta v^2}\right) \cdot \left(\frac{1}{x}\right) \leq \Delta t \leq \Delta t_{max} = \frac{x}{v}$$

For the sake of illustration, experimental searches of few meters can be conducted with a concentration of pheromone at the source equals to 1,000ng.ml$^{-1}$ with a 1ml deposit on a filter paper, ensuring minimal EAG values of 300$\mu$V up to 10 meters from the source. Still for the sake of illustration, the range of plume duration can vary from few milliseconds to 10s.

Figure 2 illustrates an example of a printed circuit board for amplifying a signal issued from an insect antenna or from another biological material that is adapted to detect odor.

The isolated antenna (or piece of biological material) is connected from both ends, via a connector 200, to an instrument amplifier 205, for example an instrument amplifier of the AD623N type (the AD623N instrument amplifier being made by Analog Device). According to a particular embodiment, the antenna tip is connected to the positive input that is itself connected to a Vcc/2 voltage. The other end of the antenna is connected to the negative input of the instrument amplifier.

As illustrated, both ends of the antenna are connected to a capacitor 210 that value can be, for example, 0.1 $\mu$F.

The gain of the instrument amplifier 205 is set as a function of the value of resistor 215. For example, a value of 5.44k$\Omega$ sets the gain to approximately 20.

According to the illustrated example, the reference value is set to the Vcc/2 voltage, the positive power pin is set to Vcc, and the negative power pin is set to the ground.

It is to be noted that the slow rate of signal variation and the low voltage input of the used battery (e.g. 3,7V) induce a strong capacitive coupling through the instrument amplifier and there is common mode leak through the amplifier.

The signal issued from the instrument amplifier 205 is then amplified and band-path filtered through an operational amplifier 220, for example an operational amplifier of the MC33078P type (the MC33078P operational amplifier being made by Motorola) having the high-pass cutoff frequency f=0.034Hz and low-pass frequency f=7.95Hz with a static gain G=101. Similarly to the instrument amplifier 205, there is capacitive coupling though the operational amplifier and slow voltage fluctuation (~1 min) of 10-20mV amplitude.

As illustrated, the output pin of the instrument amplifier 205 is connected to the negative input of the operational amplifier 220 via the capacitor 225 and the resistor 230 that values can be set to 47$\mu$F and 100k$\Omega$, respectively. The junction point between capacitor 225 and resistor 230 is connected to the Vcc/2 voltage via the resistor 235 that can be set to 1M$\Omega$.

According to the illustrated example, the negative input of the operational amplifier 220 is set to the Vcc/2 voltage, the positive power pin of operational amplifier 220 is set to Vcc and its negative power pin is set to the ground.

As illustrated, the antenna can be modeled as a source of voltage with a resistor 240 (e.g. a resistor having a value comprised between 0.1 and 50M$\Omega$) and a capacitor 245 (e.g. a capacitor having value comprised between 1 and 500nF) that are arranged in a parallel way.

Finally, the output signal of operational amplifier 220 is fed into micro-controller 250. Micro-controller 250 can be, for example, of the PIC12F683P type (the PIC12F683P micro-controller being made by Microchip), that is to say a 8-pin flash-based, 8-bit CMOS micro-controller with nanoWatt technology.

Micro-controller 250 runs a temporal high-pass filter to compensate occasional long time capacitive effects. Furthermore, it controls the state of led 255 or of an emitting device (not represented), preferably a wireless emitting device, that is turned on when the odor is detected and turned off otherwise, with a user-defined criterion on voltage variation. The main function that is carried out by micro-controller 250 can be seen as an integrator that output turns on or off the led 255.

The emitting device that can be used to inform on the detection of odor can be, for example, of the WiFi type or of the infrared type (WiFi is a trademark). The use of an emitting device is particularly adapted for recording the time evolution of an EAG signal.

The printed circuit board further comprises a power circuit for providing a reference voltage Vcc/2 equal to one-half the supply voltage Vcc and a virtual ground. As illustrated, such a power circuit can be based on a voltage splitter 260, for example on a voltage splitter of the TLE2426 type (TLE2426 is made by Texas Instrument), and on capacitors 265 and 270 that values can be set to 47$\mu$F and 0.1 $\mu$F, respectively.

For the sake of illustration, the battery can be a battery of the micro-avionic type, for example a battery weighting about 250 to 300mg and providing 3.7V and 8mA.h$^{-1}$. Since typical current consumption is equal to about 3mA when no odor is detected and to about 10 to 15mA when an odor is detected, the autonomy of the printed circuit board is about 30 min.

It is to be noted that to avoid erratic behavior resulting from differences between batteries, only batteries exhibiting a minimal tension decrease, when fully charge and when the led was active, are preferably used.

The weight of the printed circuit board, comprising the battery, is about 4g and can be reduced to about 1.3g in a CMS version (surface mounted device).

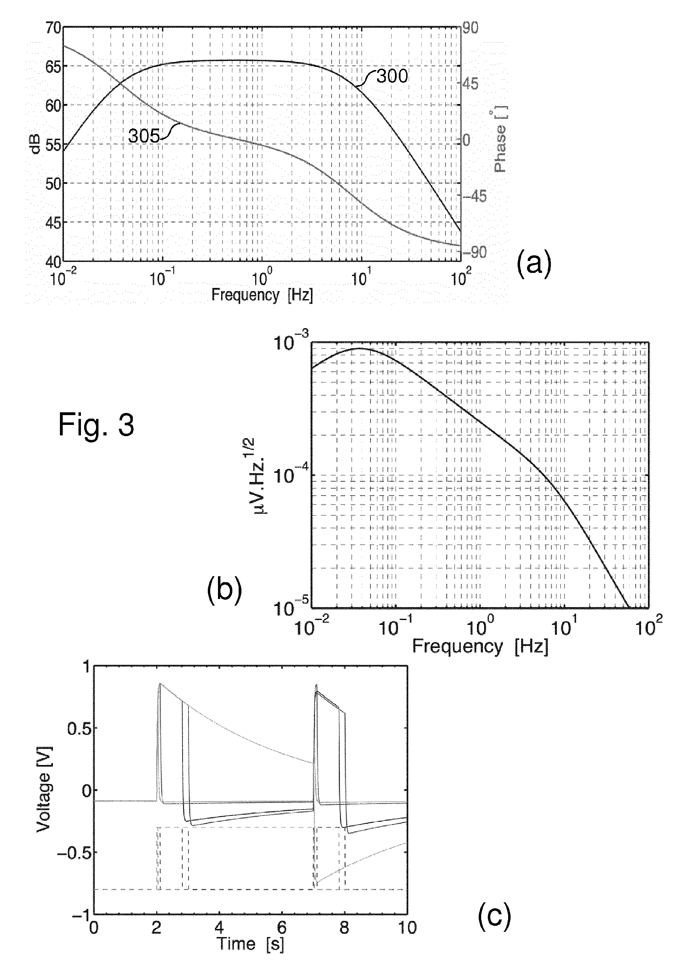

Figure 3, comprising Figures 3a, 3b, and 3C, illustrates the transfer function, the structural noise, and the pulse responses of the printed circuit board illustrated in Figure 2, respectively.

The AC signal transfer function illustrated in Figure 3a comprises an amplitude component referenced 300 and a phase component referenced 305.

The response to input pulses of various duration illustrated in Figure 3c are represented using plain lines while the signals represented with dotted lines are the input pulses (amplified 10,000 times to be visible and vertically shifted). For the sake of illustration, the pulse durations are 50ms, 100ms, 800ms, 1s, and 5s.

Experiments can be carried out to study the behavior of an animal subjected to olfactory excitation with detection of odors (e.g. a pheromone) by an insect cut antenna connected to the printed circuit board illustrated in Figure 2.

The hissing cockroach *(Gromphadorhina portentosa),* being a robust insect, is adapted to the above mentioned property according to which the detection of a pheromone plume by the cut antenna and the printed circuit board corresponds to the detection of an odor plume by the animal. The hissing cockroach is of large size (and thus, is adapted to receive a miniaturized printed circuit board), moves relatively slowly, and can be fed and attracted by yeast powder and rose petals.

*Agrotis ipsilon* is a moth whose antennae can be used as sensors. For the sake of experimentation, larvae of *Agrotis ipsilon* can be reared on an artificial diet and maintained in individual plastic cups until pupation at 23$\pm$1°C and 50$\pm$5% relative humidity. Pupae can be sexed and adult males can be kept separately from females in an inversed light/dark cycle (16h of light / 8h of dark photoperiod) at 23$\pm$1°C. Free access to a 20% sucrose solution can be given to adult moths. Experiments were performed with males, male moths being highly sensitive to their sex pheromone (the main pheromone component, cis-7-dodecenyl acetate, is the most active compound on the antenna).

An object of such experiments is to let a free animal move in an environment with a variable source of wind propagating the odor plume. Hence, the setup may mainly consist in a mostly empty room, a source of wind, here a rotating fan, a source of odor in which the pheromone corresponding to the insect antenna used as chemical sensor (e.g. moth antenna) has been added, and the moving animal (e.g. the hissing cockroach) equipped with a printed circuit board used for amplifying the signal received from the antenna, as illustrated in Figure 1 a.

For the sake of illustration, the displacement of the animal can be recorded by a camera.

To carry out an experiment, an antenna is cut close to the head of the insect (after being anesthetized, e.g. with $CO_2$). The tip is also preferably cut and both sides of the antenna are inserted into the connector of the printed circuit board. Electric contacts between the antenna and the connector can be made by using an electrophysiological solution (the quality of the signal may be improved by using chlorinated silver wires (silver wires immersed in concentrated bleach for about 20 min) but metal alloys used for classical electronics also worked efficiently).

After connecting the antenna to the printed circuit board and then the battery, the printed circuit board can be fasten to the hissing cockroach (the PCB being functional after about 30s, i.e. the time required for charging the capacitors).

The antenna of the *Agrotis ipsilon* comprises mechanical, thermo, and hygro sensors that generate a detectable EAG signal.

It is to be noted that the antenna of *Agrotis ipsilon* presents several relevant characteristics such as a mechanical robustness, high value EAG signals, an efficient EAG signal generation process with cut antenna that may last for several hours, and a relative efficiency of the moth breeding process.

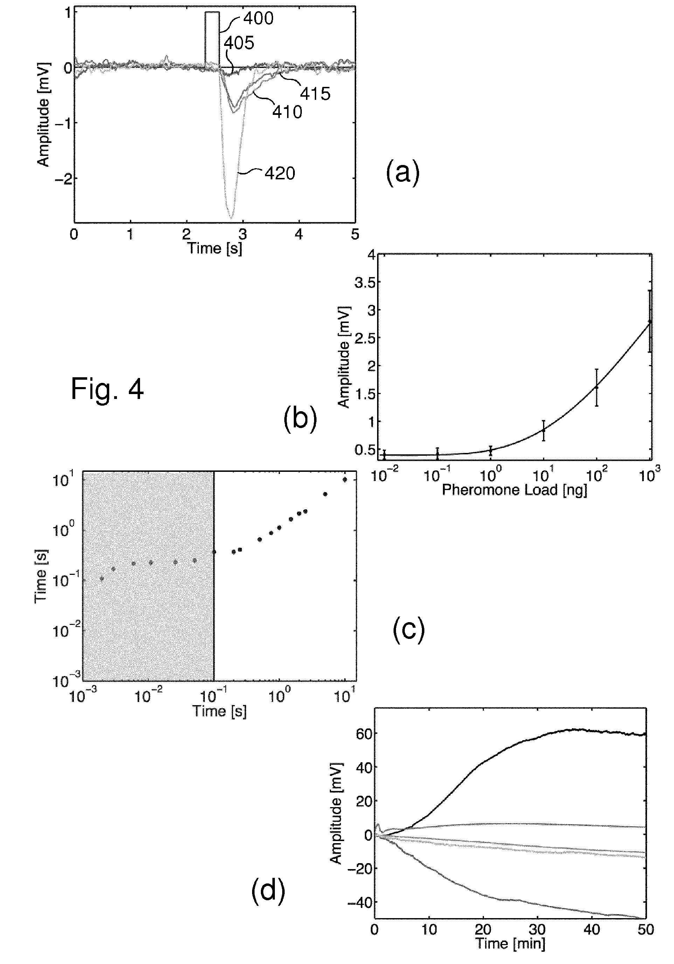

**Figure 4,** comprising Figures 4a to 4d, illustrates results obtained by carrying out experiments with a hissing cockroach provided with a printed circuit connected board to which is connected a *Agrotis ipsilon* cut antenna.

For these experiments, pheromone stimuli were delivered by blowing a humidified air pulse ($10l.h^{-1}$) through a Pasteur pipette containing a filter paper impregnated with $10\mu l$ of pheromone diluted in hexane. The tip of the Pasteur pipette was inserted in a glass tube (having a length of 20cm and an inner diameter of 7mm) positioned at 15cm from its outlet. A constant charcoal-filtered and humidified airflow was delivered on the antenna at $60l.h^{-1}$. The duration of the stimulus can be controlled by a computer-piloted electro-valve.

Stimulus concentrations refer to the quantity of pheromone applied on the filter paper. The EAG signal allows measuring the duration and concentration of odor and/or pheromone stimuli over several decades of concentration and over a large variation of stimulus durations. Non-ambiguous direct measurement of plume duration from the EAG signal is generally limited to plumes lasting more than 0.1s.

Insect's antennae are very precise and highly dynamic sensors that can match the highly variable characteristics of the turbulent plume dynamics at various distances from the source. Furthermore, the EAG signal response is more reliable than individual neuron responses. Although there are variations from one antenna to another, the global dynamics of an EAG signal is stable between insects. It is to be noted that EAG signals being easy to measure, non-functional antennae are rapidly detected and discarded.

The antenna can be approximately modeled as a resistor of 0.1 to $50M\Omega$ arranged in parallel to a capacitor which value can vary from 1 to 500nF. Similarly, the measuring electrode can be modeled as a resistor arranged in parallel with a capacitor for the systems used experimentally. The antenna variability does not prevent reliable functioning of the board. Furthermore, it has been observed that each antenna generates a significant DC component (largely superior to the EAG signal) taking values that are typically comprised between -60 to 60mV slowly varying with time as illustrated in Figure 4d. These variations are sufficiently slow (about 10min) not to be mixed with olfactory signals and are eliminated by the filters in the board.

Figure 4a illustrates the electroantennogram signal obtained in response to various concentrations of the cis-7-dodecenyl acetate pheromone. Reference 400 designates the simulation result and references 405, 410, 415, and 420 represent the measured results for pheromone concentrations of 1, 10, 100, and 1,000 ng, respectively.

It is to be noted that the high values of the EAG voltage over several decades of pheromone concentration allow sure detection of signals at larges distances from the source. However, the evolution of the curve prevents reliable inference of the pheromone concentration form the EAG amplitude or with a significant margin of error.

Figure 4b illustrates the relationship between the peak response of the EAG signal and the pheromone concentration. The dots are results of experiments and the line is spline interpolation.

Figure 4c illustrates the relationship between the duration of the detectable EAG signal corresponding to odor detection and the duration of the stimulation (100ng of pheromone). The grey area (left hand side) corresponds to stimulation duration that cannot be clearly deduced from the detectable EAG duration. It also corresponds to the low limit of pheromone plume duration in experimental conditions.

Figure 4d represents the evolution of the EAG signal DC component over time (in minutes) without stimulation. Each curve corresponds to a particular antenna. It is to be noted that the slow evolution of the DC component prevents anomalous pheromone plume detection.

As it results from the previous described experiments, it is possible to measure the detection of odor from a moving animal or a moving apparatus by using an insect antenna from another animal as a detector and an electronic board to amplify the signal. The use of an antenna from another insect species allows choosing the concentration of the pheromone to ensure detections at large distances. Interestingly, reliable electrophysiological measures can be performed without electromagnetic protections even for very slow input signals. It is the consequence of the high quality signal generated by the antenna.

Surprisingly, this biological sensor can last for one hour with constant sensitivity and for several hours with decreasing sensitivity if both ends of the antennae do not dry out.

It is to be noted that insects, living in various environments, are bound to have antennae with various properties such as response times, amplitudes, and robustness. Accordingly, depending on the needs, one might choose the antenna to be used as a function of the antenna properties.

For the sake of illustration, the response time of the antennae of the Red Palm Weevil (RPW) *Rhynchophorus ferrugineus* to its aggregation pheromone ((4S,5S)-4-methylnonan-5-ol) is much longer and has a larger amplitude than the ones of *Agrotis ipsilon.* Therefore, it is of interest to use Red Palm Weevil's antenna for large distance needs, where there would be a strong decrease in pheromone concentration and where long duration detections are expected. The high amplitude of the EAG signal would also allow significant reduction of the board amplification thus reducing capacitive coupling. Conversely, the EAG signal response of the Egyptian Cotton Leafworm (ECL) *Spodoptera littoralis* is inferior to the ones of *Agrotis Ipsilon* but more reproducible. The duration of the response scales as the duration of the stimuli.

According to a particular embodiment, the method and device for measuring odor detection is combined with a system that aims at searching for an emitting source in a turbulent flow, lacking space perception and cognitive and/or probabilistic maps, allowing effective searches, in complex varying environments, with limited access to cues, by using a searching system having limited space processing capacities.

The emitting source can be, for example, a source of particles, molecules, or fragments of molecules, a heat source, or any other source of which measurements of emission propagation at locations different from that of the emitting source can be obtained from those locations.

As mentioned above, a specific odor, typically a specific pheromone, chosen as a function of the antenna connected to the printed circuit board that is used, is mixed with particles, molecules, or fragments of molecules that source is searched for or emitted from a source located in the vicinity of the sources that is searched for.

According to a particular embodiment, the searching system comprises a processing device, an amplifier board coupled to a chemical sensor for detecting a specific odor, and moving means such as motorized wheels.

All the elements of the searching system can be integrated in one moving apparatus, like an active mobile robot (that is to say an "intelligent" mobile robot), or can be integrated as two or more apparatus communicatively coupled. For the sake of illustration, the searching system may comprise a static computer, for example of the PC *(Personal Computer)* type, provided with a communication interface, and a passive mobile robot (that is to say a remotely controlled mobile robot) comprising the amplifier board coupled to the chemical sensor, a communication interface that is compatible with that of the computer, and motorized wheels controlled by the computer. The communication interfaces may comply with any communication standard, preferably a wireless communication standard such as the one known as ISO/CEI 8802-11. The active or passive mobile robot can move along a self-generated path, with or without user-defined constraints, to locate the diffusing source.

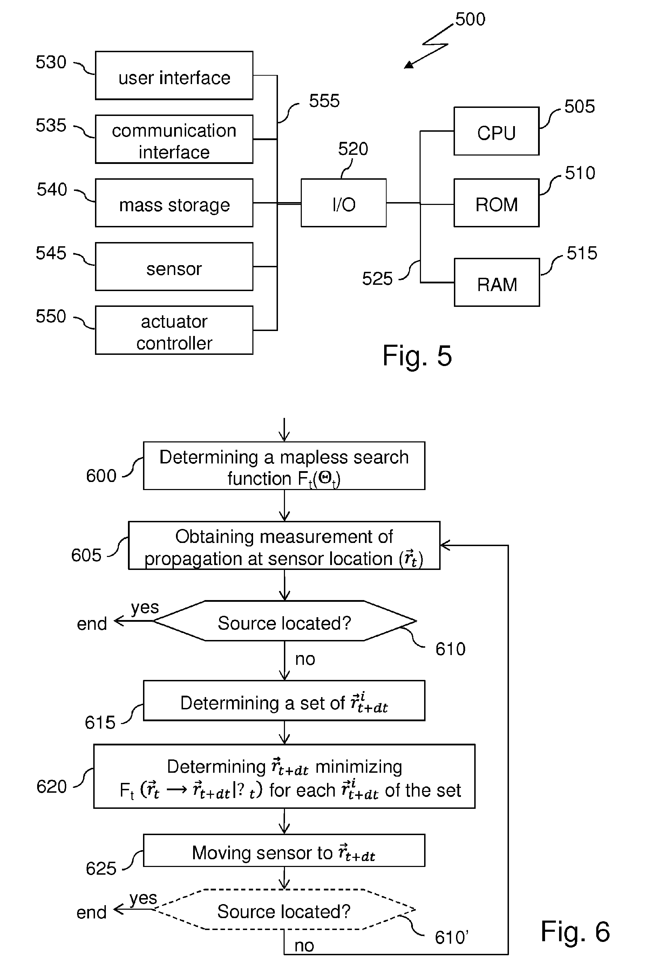

**Figure 5** illustrates an example of the architecture of a searching system 500. As shown, the searching system 500 comprises a central processing unit (CPU) 505, a Read-Only Memory (ROM) 510, a Random Access Memory (RAM) 515, and an input/output (I/O) subsystem 520, all of them being connected to a system bus 525. The I/O subsystem 520 may include one or more controllers for input/output devices such as user interface 530 (which may comprise a keyboard, a cursor control device, and a display device), communication interface 535 (which can be of wire or wireless type), mass storage device 540, at least one amplifier board coupled to a chemical sensor 545 for remotely obtaining emission propagation measurements of an element associated with the emitting sources searched for, and actuator controller 550 for controlling displacements of the searching system (or at least of the sensor 545).

Depending upon the application of the searching system 500, one or more further I/O devices may be connected to the I/O subsystem 520. Typically, the hardware system 500 is controlled by an operating system that can be stored in ROM 510 or in mass storage device 545, which in turn controls various tools and applications that are generally loaded in RAM 515.

The searching system may include several types of amplifier boards and chemical sensors to localize several kinds of emitting sources and/or to analyze particular features of the environment e.g., detecting obstacles.

Some of the elements of the searching system such as CPU 505, ROM 510, RAM 515, I/O subsystem 520, all of them being connected to a system bus 525, user interface 530, communication interface 535, and/or mass storage device 540 may be part of a generic computer device, handheld device, or any kind of computer device.

According to a particular embodiment, the searching system is based on the use of the projection of a probability of the emitting source position into a standardized form, to avoid the need of advanced space perception, and on the evaluation by the searching system (of which the position of the amplifier board and chemical sensor is known from uncorrected noisy cueless path integration) of a free energy that is minimized in order to find the right path to the source.

A parameter referred to as the "internal temperature" allows an efficient balance between exploration of the search space and exploitation of information already obtained.

**Figure 6** illustrates steps of an algorithm to search for an emitting source of which measurements of emission propagation at locations different from that of the emitting source can be obtained from those locations, in a highly variable environment, lacking space perception, according to an embodiment.

A first step (step 600) aims at determining a mapless search function $F_t(\Theta_t)$ representing a free energy that is to be minimized in order to find the right path to the source. An example of such a mapless search function is described herein below.

In a next step (step 605), a measurement of emission propagation related to the emitting source, at the location of the amplifier board and of the chemical sensor (referred to as the sensor location here after), is obtained.

Such a measurement is typically a value, preferably a normalized value, for example a value varying from zero to one hundred, or a binary value, for example a binary value of which the value zero indicates that no emission propagation characteristic of the emitting source has been detected and the value one indicates that an emission propagation characteristic of the emitting source has been detected. It is to be noted that a binary value may be obtained from another type of value by using appropriate thresholds.

As mentioned above, this measurement can be directed to the particles, molecules, or fragments of molecules that source is searched for or can be directed to molecules, typically pheromones, mixed with the particles, molecules, or fragments of molecules that source is searched for or emitted from a source located in the vicinity of the sources that is searched for.

Next, according to a particular embodiment, a test is performed to determine whether or not the emitting source is located at sensor location $\vec{r}_t$ (step 610). Such a test can be performed, for example, by comparing the measured value with a threshold or by comparing a detection rate with a threshold.

Once a measurement of emission propagation related to the emitting source, at sensor location $\vec{r}_t$, has been obtained, a set of next possible sensor locations $\vec{r}^i_{t+dt}$ is determined (step 615). According to a particular embodiment, a set of next possible sensor relative locations are predetermined. For the sake of illustration, such a set of next possible sensor relative locations can be set as follows:

$$\vec{r}^1_{t+dt}\left(x_{\vec{r}_t} + 1, y_{\vec{r}_t}\right)$$

$$\vec{r}^2_{t+dt}\left(x_{\vec{r}_t} - 1, y_{\vec{r}_t}\right)$$

$$\vec{r}^3_{t+dt}\left(x_{\vec{r}_t}, y_{\vec{r}_t} + 1\right)$$

$$\vec{r}^4_{t+dt}\left(x_{\vec{r}_t}, y_{\vec{r}_t} - 1\right)$$

$$\vec{r}^5_{t+dt}\left(x_{\vec{r}_t} + 1, y_{\vec{r}_t} + 1\right)$$

$$\vec{r}^6_{t+dt}\left(x_{\vec{r}_t} - 1, y_{\vec{r}_t} + 1\right)$$

$$\vec{r}^7_{t+dt}\left(x_{\vec{r}_t} + 1, y_{\vec{r}_t} + 1\right)$$

$$\vec{r}^{8}_{t+dt}\left(x_{\vec{r}_t} - 1, y_{\vec{r}_t} - 1\right)$$

Next, a variation $\Delta F_t$ of the free energy as defined by the predetermined mapless search function $F_t(\Theta_t)$ between sensor location $\vec{r}_t$ and each of the next possible sensor locations $\vec{r}^{i}_{t+dt}$ is computed. The result having the minimum value is selected to determine the next sensor location $\vec{r}_{t+dt}$ (step 620).

According to a particular embodiment, the sensor may stay at its previous location $\vec{r}_t$ if, for example, the minimum variation $\Delta F_t$ of the free energy exceeds a predetermined threshold.

After having being determined, the sensor is moved to the next sensor location $\vec{r}_{t+dt}$ (step 625) and the algorithm is branched to step 605 to obtain a new measurement of emission propagation related to the emitting source, at a sensor location, and to repeat the previous described steps until the emitting source is reached. The obtained measurement of emission propagation is advantageously memorized along with current sensor location $\vec{r}_t$ to be later used for determining a direction to follow.

According to a particular embodiment, test 610 performed to determine whether or not the emitting source is located at sensor location $\vec{r}_t$ may be replaced by test 610' that aims at determining whether or not the emitting source is located at location $\vec{r}_{t+dt}$. Such a test may consist in comparing the value of $F_{t+dt}(\Theta_t)$ with a predetermined threshold. As described herein above, function $F_t(\Theta_t)$ can be chosen so that $F_{t+dt}(\Theta_t) = 1$ if the source is at location $\vec{r}_{t+dt}$. Such a test may also be based on another type of measurements.

As described above, the searching system moves along a self-generated path denoted $\Theta_t$ (with or without user-defined constraints) and, at each iterating step, determines a direction to follow based on the events experienced at previous location of path $\Theta_t$. The searching system accesses its position from uncorrected path integration and is considered to evolve in a cue-limited environment preventing an accurate correction of its position. According to this particular embodiment, information bearing events are based on measurements of emission propagation of the emitting source at the sensor location. For the sake of example, it can be based on either detections of molecules (rare local events) or the absence of detection of molecules (frequent less localized events). Measurements of emission propagation can be compared to one or more thresholds, for example if the measurements represent particle density, to result in detections or absences of detection.

It is to be noted that the environment may be characterized by a function denoted $R(\vec{r}|\vec{r}_0)$ representing the rate of detection, which gives a rate of detection at location $\vec{r}$ when the source is located at location $\vec{r}_0$. A simplified model of such a function $R(\vec{r}|\vec{r}_0)$ can be, for example, a simple Gaussian function. In the case where the diffusing source is a molecule diffusing source in the air, the exact expression reads as follows:

$$R\left(\vec{r}|\vec{r}_0\right) = \frac{R}{\ln\left(\dfrac{\lambda}{a}\right)} e^{-\left(\frac{(y-y_0)V}{2D}\right)} K_0\left(\frac{|\vec{r} - \vec{r}_0|}{\lambda}\right)$$

where
$\tau$ and R represent the lifetime of emitted molecules and their emission rate, respectively;
$\vec{V}$ represents a mean wind in the environment;
$a$ is the characteristic length of the molecule detector;
$K_0$ is the modified Bessel function of order zero;
(y and $y_0$) represents where the wind was taken to blow in the y direction and $y_0$ is the y-coordinate of the diffusing source; and
$\lambda$ is defined as follows:

$$\lambda = \sqrt{\frac{D\tau}{1 + \dfrac{V^2\tau}{4D}}}$$

where D represents the coefficient of diffusion of the molecules.

The absence of complex space perception prevents the direct decoding by inference of the information stored in the path $\Theta_t$ using Bayesian inference. Hence, all terms involved in the path choosing mechanism have to be expressed in a standardized form to be computed without the complete expression of the time varying probability of finding the source at a particular location. The following standardized projected probability can be used,

$$P_t^M\left(\vec{r}_0\middle|\Theta_t\right) = \frac{e^{\frac{\|\vec{r}_0-\vec{r}_G\|^2}{\lambda_G^2}}\left(1 - \frac{1}{N_M}\sum_{j=N_t-N_M+1}^{N_t} e^{\frac{\|\vec{r}_0-\vec{r}_j\|^2}{\lambda_u^2}}\right)}{Z_t}$$

where

$P_t^M(\vec{r}_0|\Theta_t)$ is the probability that the emitting source is located in $\vec{r}_0$ knowing path $\Theta_t$;

$\vec{r}_G$ represents the damped mass center of detections and $\lambda_G$ represents the scale associated with the Gaussian term approximating the detection terms;

$\vec{r}_j$ represents the locations of the searching system sensor where no detection has been made;

$N_M$ is the number of measurement locations;

$\lambda_u$ represents the scale of the Gaussian terms approximating the non-detection terms; and

$Z_t$ is a normalization constant.

The lack of space perception coupled with the accumulation of positioning errors lead, preferably, to precautions being taken in relation to the amount of "memory" that the searching system should keep from its past. Hence, the damped center of mass can be expressed as follows:

$$\vec{r}_G = \frac{\sum_{i=1}^{G} \gamma^{G-i}\vec{r}(t_i)}{\sum_{i=1}^{G} \gamma^{G-i}}$$

where $\gamma<1$ and $\{t_i, i=1..G\}$ represents the times of the measurements.

As described above, the searching system is based on the use of the projection of a probability of the diffusion source position into a standardized form and on the evaluation of a free energy. Accordingly, to decide the direction to be taken after experiencing the self-generated path $\Theta_t$ (in view of user-defined constraints if any), a function of $P_t^M\left(\vec{r}_0\middle|\Theta_t\right)$ is to be determined. The minimum value obtained from such a function of $P_t^M\left(\vec{r}_0\middle|\Theta_t\right)$, according to one of a plurality of possible directions, is used to choose the direction to be taken.

Optimization based on entropy computation is known to be efficient (in particular, from EP 1,881,389). However, since it has been observed that entropy tends to shift the exploration versus exploitation balance towards exploration, another term has been added to reinforce maximum likelihood behavior and so shift the balance towards exploitation. Accordingly, a free energy can be expressed according to the following formula:

$$F_t(\Theta_t) = W_t(\Theta_t) + TS(\Theta_t)$$

where

$W_t(\Theta_t)$ represents a working energy;

$S(\Theta_t)$ represents the Shannon entropy; and

T a temperature parameter that controls the relative value between these two elements.

Therefore, the free energy can be expressed as follows:

$$F_t(\Theta_t) = \iint_{|\vec{r}_0 - \vec{r}_G| \leq \frac{\lambda_G}{2}} d\vec{r}_0 P_t^M(\vec{r}_0|\Theta_t) + T\left[-\iint d\vec{r}_0 P_t^M(\vec{r}_0|\Theta_t) \log P_t^M(\vec{r}_0|\Theta_t)\right]$$

When moving from the sensor position at time t, denoted $\vec{r}_t$, to a neighboring position corresponding to time t+dt, denoted $\vec{r}_{t+dt}$, the expected variation of the free energy is the sum of two terms, one accounting for the discovery of the source and the other one for its absence. This variation reads as follows:

$$\Delta F_t\left(\vec{r}_t \rightarrow \vec{r}_{t+dt}|\Theta_t\right) = P_t^M\left(\vec{r}_{t+dt}\right)\Delta F_t^{discovery} + \left(1 - P_t^M\left(\vec{r}_{t+dt}\right)\right)\Delta F_t^{nothing}$$

$$= P_t^M\left(\vec{r}_{t+dt}\right)\left[1 - F_t(\Theta_t)\right] + \left(1 - P_t^M\left(\vec{r}_{t+dt}\right)\right)\left[\rho_0\left(\vec{r}_{t+dt}\right)\Delta F_t^0\left(\Theta_t\right) + \rho_1\left(\vec{r}_{t+dt}\right)\Delta F_t^1\left(\Theta_t\right)\right]$$

where

$P_t^M\left(\vec{r}_{t+dt}\right)$ represents the probability that the source is at position $\vec{r}_{t+dt}$;

$F_t(\Theta_t)$ represents the free energy of the searcher at time t;

$F_{t+dt}(\Theta_t) = 1$ is the free energy if the source is found ;

$\left[1 - P_t^M\left(\vec{r}_t\right)\right]$ is the probability that the source is not at position $\vec{r}_{t+dt}$ ;

$\rho_i(\vec{r}_{t+dt})$ represents the expected probability of having i detections; and $\Delta F_t^i\left(\Theta_t\right)$ is the expected free energy variation if there were i detections.

According to the Poisson detection function, the expected probability of having i detections can be expressed as follows:

$$\rho_i\left(\vec{r}\right) = \frac{\left[h\left(\vec{r}\right)\right]^i e^{-h(\vec{r})}}{i!}$$

where $h(\vec{r})$ represents the expected average hit rate that can be expressed as follows:

$$h\left(\vec{r}\right) = \int d\vec{r}_0 R\left(\vec{r}|\vec{r}_0\right) P_t^M\left(\vec{r}_0\right)$$

where $R(\vec{r}|\vec{r}_0)$ is the rate of detection of the searching system if it is at the position $\vec{r}$ and the source is at $\vec{r}_0$. This can be approximated using a Gaussian function.

The updating rule of $\vec{r}_G$, to avoid storing the hit positions, can be expressed as follows:

$$\vec{r}_{G+1} = \gamma \frac{1 - \gamma^G}{1 - \gamma^{G+1}} \vec{r}_G + \frac{1 - \gamma}{1 - \gamma^{G+1}} \vec{r}\left(t_{G+1}\right)$$

In case of turbulent transport (e.g. wind), the updating procedure of $\vec{r}_G$ can be expressed as follows:

$$X_G = \frac{\sum_{i=1}^{G} \gamma(V)^{G-i} x(t_i)}{\sum_{i=1}^{G} \gamma(V)^{G-i}}$$

and

$$y_G = y(t_G)$$

with the turbulent transport being oriented in the -y (minus y) direction (at time t), e.g. wind blowing in the -y direction, and $\gamma(V)$ is the damping factor decreasing with rising velocity.

Depending on the characteristics of the environment where the emitting source is to be found, the shape of $\gamma(V)$ may be chosen to better fit the characteristics of the detection. Linear dependency with a maximum velocity is found to be efficient. Obviously, if the turbulent transport direction varies with a similar time scale as the sensor is moved, the search becomes extremely difficult, and the updating procedure of $\vec{r}_G$ is preferably the one used in the windless scheme.

It is to be noted that in order to ensure that an emitting source will be found, the drift velocity information is underexploited. The symmetry of the distribution ensures that the searcher can go in the opposite direction to the turbulent transport (e.g. wind) if it has moved beyond the source position.

Drift velocity information processing can be improved as follows: after each detection, a random distance L is randomly generated from an exponential distribution of correlation length $L_T = \chi \lambda_e$ ( $\chi$ being user-defined) and the sensor is moved according to distance L in the opposite direction to the turbulent transport such as wind (at the time). Conversely, to reinforce stability of the search for the rare (<1/500) searches where a sequence of detection with low probabilities happened (far from the source), the sensor is moved back to the last point (with the accumulated odometry error) where a detection has been made after a user-defined time without any detection. Finally, initial spiraling is made on an asymmetrical spiral, which is more extended when going in the opposite direction to the turbulent transport (e.g. wind) than when going in the direction of the turbulent transport.

If the turbulent transport is expected not to change direction (or to change direction only slightly) during the search, asymmetry between turbulent transport direction and perpendicular direction can be added to $P_t^M\left(\vec{r}_0 \middle| \Theta_t\right)$ to reinforce turbulent transport information use.

If the constraint of always finding the source is removed, the efficiency of the search for the source can be improved. For example, reducing the choice of possible moving points to the ones in the opposite direction to the turbulent transport (e.g. wind) and diminishing the internal temperature T to reinforce information exploitation leads to fast searches when the searcher finds the source.

It is to be noted that all the terms involved in determining the direction to be taken after experiencing the self-generated path $\Theta_t$, i.e. $F_t(\Theta_t)$, $W_t(\Theta_t)$, $S(\Theta_t)$, $h_t(\vec{r}_t)$, and $\rho_t(\vec{r}_t)$, can be approximated so that they do not depend on $P_t^M\left(\vec{r}_0 \middle| \Theta_t\right)$, for example according to the following formulae:

$$W(\Theta_t) = \iint_S d\vec{r}_0 P_t(\vec{r}_0|\Theta_t) = \frac{1}{Z_t}\left[\pi\left(erf\left(\frac{1}{2}\right)\lambda_G\right)^2 - \frac{1}{N_M}\sum_{j=N_t-N_M+1}^{N_t}\frac{\pi\lambda_G^2\lambda_u^2}{4\left(\lambda_G^2+\lambda_u^2\right)}e^{-\frac{\|\vec{r}_G-\vec{r}_j\|^2}{\left(\lambda_G^2+\lambda_u^2\right)}}\Phi\left(x_j, x_G\right)\Phi\left(y_j, y_G\right)\right]$$

with S representing the integration domain that is defined here by $|\vec{r}_0 - \vec{r}_G| \leq \frac{\lambda}{2}$ and where

$$\Phi\left(x_j, x_G\right) = erf\left(\frac{-\lambda_d^2 - 2x_j\lambda_d + 2x_G\lambda_d - \lambda_u^2}{2\lambda_u\sqrt{\lambda_G^2 + \lambda_u^2}}\right) - erf\left(\frac{\lambda_d^2 - 2x_j\lambda_d + 2x_G\lambda_d + \lambda_u^2}{2\lambda_u\sqrt{\lambda_G^2 + \lambda_u^2}}\right)$$

$$S(\Theta_t) \approx \log(Z_t) + \frac{\pi}{2Z_t}\left(\lambda_G^2 + \lambda_u^2\right) + \frac{\pi}{Z_t}\sum_{j=N_t-N_M+1}^{N_t} e^{-\left(\frac{\|\vec{r}_G-\vec{r}_j\|^2}{\lambda_G^2+\lambda_u^2}\right)}\left[\frac{\lambda_G^2\lambda_u^2}{\left(\lambda_G^2+\lambda_u^2\right)} - \frac{\lambda_G\lambda_u\left(\lambda_G^3\lambda_u\left(x_G-x_i\right)^2 + \lambda_G\lambda_u^3/2\right)}{\left(\lambda_G^2+\lambda_u^2\right)^2} - x \to y\right]$$

with the approximation $\log\left(1 - \frac{1}{N_M}\sum_{j=N_t-N_M+1}^{N_t} e^{-\frac{\|\vec{r}_0-\vec{r}_j\|^2}{\lambda_u^2}}\right) \approx -\frac{1}{N_M}\sum_{j=N_t-N_M+1}^{N_t} e^{-\frac{\|\vec{r}_0-\vec{r}_j\|^2}{\lambda_u^2}}$ and where all the cross terms $\frac{1}{N_M}\sum_{j=N_t-N_M+1}^{N_t} e^{-\frac{\|\vec{r}_0-\vec{r}_j\|^2}{\lambda_u^2}} \times \frac{1}{N_M}\sum_{j=N_t-N_M+1}^{N_t} e^{-\frac{\|\vec{r}_0-\vec{r}_j\|^2}{\lambda_u^2}}$ have been neglected (mean field approximation); and

$$h(\vec{r}) = \frac{\pi\kappa}{Z_t}\left(\frac{1}{2}\lambda_G^2 e^{-\frac{\|\vec{r}_G-\vec{r}\|^2}{2\lambda_G^2}} - \frac{1}{N_M}\sum_{j=N_t-N_M+1}^{N_t}\frac{\lambda_G^2\lambda_u^2}{\left(\lambda_G^2+2\lambda_u^2\right)}\Psi\left(x_G, x_j, x\right)\Psi\left(y_G, y_j, y\right)\right)$$

with the user defined factor $\kappa$ and $\Psi\left(x_G, x_j, x\right) = e^{\frac{\left(-\left(x_G^2+x^2\right)\left(\lambda_G^2+\lambda_u^2\right) + \lambda_G^2\left(-2x_j^2 + 2x_G x_j + 2xx_j\right) + 2x_G x\lambda_u^2\right)}{\left(\lambda_G^2+2\lambda_u^2\right)\lambda_G^2}}$ .

It is also to be noted that such formulae can be expressed differently to optimize updates and to reduce required memory accesses. To that end, the standardized form of the probability field can be expressed as follows:

$$P_t^M\left(\vec{r}_0\,|\,\Theta_t\right) = \frac{e^{-\frac{\|\vec{r}_0-\vec{r}_G\|^2}{\lambda_G^2}}\left(1 - \sum_{j=1}^{N_t}\rho^{N_t-j} e^{-\frac{\|\vec{r}_0-\vec{r}_j\|^2}{\lambda_u^2}}\right)}{Z_t} \text{ with } \rho < 1$$

By defining $\Xi_t\left(\vec{r}_0\,|\,\Theta_t\right)$ as follows:

$$\Xi_t\left(\vec{r}_0\,|\,\Theta_t\right) = \sum_{j=1}^{N_t}\rho^{N_t-j} e^{-\frac{\|\vec{r}_0-\vec{r}_j\|^2}{\lambda_u^2}}$$

an updating rule may be expressed as follows:

$$\Xi_{t+dt}\left(\vec{r}_0 \middle| \Theta_{t+dt}\right) = \rho \Xi_t\left(\vec{r}_0 \middle| \Theta_t\right) + e^{-\frac{\left(\vec{r}_0 - \vec{r}_{N+1}\right)^2}{\lambda_u^2}}$$

Similarly by defining $Z_t$ as follows:

$$Z_t = \pi\lambda_G^2 - \Gamma_t$$

with

$$\Gamma_t = \pi \frac{\lambda_G^2 \lambda_u^2}{\lambda_G^2 + \lambda_u^2} \sum_{j=1}^{N_t} \rho^{N_t - j} e^{-\frac{\left|\vec{r}_G - \vec{r}_j\right|^2}{\lambda_G^2 + \lambda_u^2}}$$

and the following updating rules

$$\Gamma_{t+dt} = \rho\Gamma_{t+dt} + \pi \frac{\lambda_G^2 \lambda_u^2}{\lambda_G^2 + \lambda_u^2} e^{-\frac{\left|\vec{r}_G - \vec{r}_{N+1}\right|^2}{\lambda_G^2 + \lambda_u^2}}$$

hence

$$Z_{t+dt} = \pi\lambda_G^2 - \Gamma_{t+dt}.$$

Furthermore, the work energy becomes

$$W\left(\Theta_t\right) = \frac{1}{Z_t}\left[\pi\left(erf\left(\frac{1}{2}\right)\lambda_G\right)^2 - \sum_{j=1}^{N_t} \rho^{N_t - j} \frac{\pi\lambda_G^2 \lambda_u^2}{4\left(\lambda_G^2 + \lambda_u^2\right)} e^{-\frac{\left|\vec{r}_0 - \vec{r}_G\right|^2}{\lambda_G^2 + \lambda_u^2}} \Phi\left(x_j, x_G\right)\Phi\left(y_j, y_G\right)\right]$$

By defining

$$\Omega_t = \sum_{j=1}^{N_t} \rho^{N_t - j} \frac{\pi\lambda_G^2 \lambda_u^2}{4\left(\lambda_G^2 + \lambda_u^2\right)} e^{-\frac{\left|\vec{r}_j - \vec{r}_G\right|^2}{\lambda_G^2 + \lambda_u^2}} \Phi\left(x_j, x_G\right)\Phi\left(y_j, y_G\right)$$

and the updating rule

$$\Omega_{t+dt} \leftarrow \rho\Omega_t + \frac{\pi\lambda_G^2\lambda_u^2}{4\left(\lambda_G^2 + \lambda_u^2\right)} e^{-\frac{|\vec{r}_{N+1}-\vec{r}_G|^2}{\lambda_G^2+\lambda_u^2}} \Phi\left(x_{N+1}, x_G\right)\Phi\left(y_{N+1}, y_G\right)$$

hence

$$W\left(\Theta_{t+dt}\right) = \frac{1}{Z_{t+dt}}\left[\pi\left(erf\left(\frac{1}{2}\right)\lambda_G\right)^2 - \Omega_{t+dt}\right]$$

and

$$S\left(\Theta_t\right) \approx \log(Z_t) + \frac{\pi}{2Z_t}\left(\lambda_G^2 + \lambda_u^2\right) +$$

$$\frac{\pi}{Z_t}\sum_{i=1}^{N_t}\rho^{N_t-i}e^{-\frac{|\vec{r}_G-\vec{r}_i|^2}{\lambda_G^2+\lambda_u^2}}\left[\frac{\lambda_G^2\lambda_u^2}{\lambda_G^2+\lambda_u^2} - \frac{\lambda_G\lambda_u\left(\lambda_G^3\lambda_u\left(x_G-x_i\right)^2 + \lambda_G\lambda_u^3/2\right)}{\left(\lambda_G^2+\lambda_u^2\right)^2} - \frac{\lambda_G\lambda_u\left(\lambda_G^3\lambda_u\left(y_G-y_i\right)^2 + \lambda_G\lambda_u^3/2\right)}{\left(\lambda_G^2+\lambda_u^2\right)^2}\right]$$

with

$$\Psi_t = \sum_{i=1}^{N_t}\rho^{N_t-i}e^{-\frac{|\vec{r}_G-\vec{r}_i|^2}{\lambda_G^2+\lambda_u^2}}\left[\frac{\lambda_G^2\lambda_u^2}{\lambda_G^2+\lambda_u^2} - \frac{\lambda_G\lambda_u\left(\lambda_G^3\lambda_u\left(x_G-x_i\right)^2 + \lambda_G\lambda_u^3/2\right)}{\left(\lambda_G^2+\lambda_u^2\right)^2} - \frac{\lambda_G\lambda_u\left(\lambda_G^3\lambda_u\left(y_G-y_i\right)^2 + \lambda_G\lambda_u^3/2\right)}{\left(\lambda_G^2+\lambda_u^2\right)^2}\right]$$

the updating rule

$$\Psi_{t+dt} = \rho\Psi_{t+dt} + e^{-\frac{|\vec{r}_G-\vec{r}_{N+1}|^2}{\lambda_G^2+\lambda_u^2}}\left[\frac{\lambda_G^2\lambda_u^2}{\lambda_G^2+\lambda_u^2} - \Theta\left(x_G, x_{N+1}\right) - \Theta\left(y_G, y_{N+1}\right)\right]$$

with

$$\Theta\left(x_G, x_{N+1}\right) = \frac{\lambda_G\lambda_u\left(\lambda_G^3\lambda_u\left(x_G-x_{N+1}\right)^2 + \lambda_G\lambda_u^3/2\right)}{\left(\lambda_G^2+\lambda_u^2\right)^2}$$

leading to

$$S\left(\Theta_{t+dt}\right) \approx \log(Z_{t+dt}) + \frac{\pi}{2Z_{t+dt}}\left(\lambda_G^2 + \lambda_u^2\right) + \frac{\pi}{Z_{t+dt}}\Psi_{t+dt}.$$

It is to be noted that for both $\Omega_t$ and $\Psi_t$ an approximation is preferably made in the updating rule: the function $\Phi$ depends on $\vec{r}_G$ which varies in time but the updating rules assume that $\vec{r}_G$ does not vary for the terms earlier in time.

Finally,

$$h_t(\vec{r}) = \frac{\pi\kappa}{Z_t}\left(\frac{1}{2}\lambda_G^2 e^{\frac{|\vec{r}_G-\vec{r}|^2}{2\lambda_G^2}} - A_t\right)$$

with

$$A_t = \sum_{j=1}^{N_t} \rho^{N_t-j}\frac{\lambda_G^2\lambda_u^2}{2\lambda_u^2+\lambda_G^2}\Omega(x_G, x, x_{N+1})\Omega(y_G, y, y_{N+1})$$

with

$$\Omega(x_G, x, x_{N+1}) = e^{\frac{\left[-x_G^2(\lambda_u^2+\lambda_G^2)-x^2(\lambda_u^2+\lambda_G^2)+\lambda_G^2(-2x_{N+1}^2+2x_Gx_{N+1}+2xx_{N+1})+2x_Gx\lambda_u^2\right]}{(2\lambda_u^2+\lambda_G^2)\lambda_G^2}}$$

and the following updating rule

$$A_{t+dt} = \rho A_t + \frac{\lambda_G^2\lambda_u^2}{2\lambda_u^2+\lambda_G^2}\Omega(x_G, x, x_{N+1})\Omega(y_G, y, y_{N+1})$$

leading to

$$h_{t+dt} = \frac{\pi\kappa}{Z_{t+dt}}\left(\frac{1}{2}\lambda_G^2 e^{\frac{|\vec{r}_G-\vec{r}|^2}{2\lambda_G^2}} - A_{t+dt}\right)$$

Accordingly, the direction to be taken can be computed easily without huge resources.

During the search, two processes ought to be balanced: exploration of the environment to get more information on the localization of the emitting source and exploitation of the already accumulated information.

In Infotaxis, as disclosed in particular in EP 1,881,389, the entropy is used to balance exploration and exploitation. Based on the reliable inference of the source position distribution $P_t(\vec{r}_0)$, the balance is efficiently managed with a clear advantage towards exploration.

According to a particular embodiment, information accessible from $P_t^M(\vec{r}_0|\Theta_t)$ is only partially reliable since the searching system has no exact access to the sensor position and the detection process may not correspond to the one used in the model used. The use of the working energy $W_t(\Theta_t)$ allows reinforcement of the maximum likelihood behavior and the temperature T allows the balance to be shifted between exploration and exploitation. It is to be noted that both exploration and exploitation are present in the entropy $S(\Theta_t)$ where mostly exploitation is present in the working energy $W_t(\Theta_t)$. This allows the temperature to actively control the balance. Furthermore, the working energy $W_t(\Theta_t)$ prevents the self-trapping behavior observed during simulations performed for high temperatures (T>5), when a large number of detections has been made far from (rare) and close to the source.

It is to be noted that at short initial distances (between the sensor and the emitting source), the use of a low temperature value is more efficient because the working energy $W_t(\Theta_t)$, which favors maximum likelihood behavior, has more influence on the decision process than at high temperature. As the initial distance rises, the optimum temperature rises with a flattening of the mean search time-temperature curve.

According to a particular embodiment, the sensor is moved after an initial detection. The initial movement is preferably defined as an Archimedean spiral. Such an Archimedean spiral movement is preferably used until a second detection is made. In case of turbulent transport, the initial spiral is preferably asymmetrical, with the spiral being wider in the opposite direction to the turbulent transport than in its direction.

Still according to a particular embodiment, a linear motion in a random direction may be chosen if the algorithm results in a decision of not moving the sensor.

Still in a particular embodiment, the memory of the searching system that is used to store previous detections may be reset when numerous detections have been made, after a user-defined time. Such an embodiment is particularly adapted to the case in which the sensor is initially located far from the source and hence reaches the vicinity of the source with numerous detections. Memory reset allows the balance of exploration/exploitation to shift back to exploration.

According to a particular embodiment, several independent mobile sensors, for example several intelligent mobile robots, are used to locate one or several emitting sources. The architecture and the features of these mobile sensors may be similar to those described above.

If the mobile sensors are still unable to determine their position accurately in the searching space and cannot correct odometry error, they are provided with communication means so as to transmit and/or receive data, in particular measurements of emission propagation and/or relative position information. Such data can be transmitted and/or received on a regular basis whatever the relative position of robots is or on a regular basis only if the robots are close to each other.

For the sake of illustration, two robots can locate each other using ultrasound sensors, sonars, cameras, and the like. The detection can be made by using local sensors or via a central computer able to communicate with the robots. Alternatively, all the robots are provided with their mutual initial positions, these positions being therefore known at least approximately by each robot.

According to this embodiment, the mobile sensors (or robots) move along self-generated paths $\Theta_i^t$ $i \in (1..Ns)$, with $N_s$ representing the number of robots. The latter have to decide their future paths based on information accumulated into paths $\Theta_i^t$. More precisely, each of the robots, moving in a medium where localization (SLAM, Simultaneous Localization And Mapping) is either difficult or impossible, takes individual decisions based on a global path $\Theta_t$ and estimates its position from uncorrected path integration. As a consequence, all robots accumulate odometry errors as they search for the emitting source(s) and have no direct way to correct them.

The standardized projected probability that an emitting source is located in $\vec{r_0}$ knowing path $\Theta_t$ can be defined as follows:

$$P_t^M(\vec{r_0}|\Theta_t) = \frac{e^{-\frac{\left\|\vec{r_0}-\vec{r_G^{N_S}}\right\|^2}{\lambda_G^2}}\left(-\frac{1}{N_M}\sum_{k=1}^{N_S}\sum_{j=N_t-\left[\frac{(N_M+1)}{N_S}\right]}^{N_S}e^{-\frac{\left\|\vec{r_0}-\vec{r_j^k}\right\|^2}{\lambda_u^2}}\right)}{Z_t}$$

where

$\vec{r_G^{N_S}}$ represents the damped mass center of detections made by all the robots and $\lambda_G$ represents the scale associated with the Gaussian term approximating the detection terms;

$\vec{r_j^k}$ represents the locations of the robot having index k (i.e. the searching system sensor having index k) where no detection has been made;

$N_M$ is the number of measurement locations;

$\lambda_u$ represents the scale of the Gaussian terms approximating the non-detection terms; and

$Z_t$ is a normalization constant.

Again, it is noted that the lack of space perception coupled with the accumulation of positioning errors lead, preferably, to precautions being taken in relation to the amount of "memory" that the searching system should keep from its past.

Like the damped centre of mass expressed for the detections made by a single robot, the damped mass center of detections made by all the robots can be expressed as follows:

$$\overrightarrow{r_G^{N_S}} = \frac{\sum_{i=1}^{G} \gamma^{G-i} \overrightarrow{r(t_i)}}{\sum_{i=1}^{G} \gamma^{G-i}}$$

where $\gamma < 1$, $\overrightarrow{r(t_i)}$ is the position of one robot which made the detection at time $t_i$, and $\{t_i, i=1..G\}$ represents the times of the measurements ordered from the most recent detections to the oldest detections.

In such a swarm searching process, robots share information regarding their trajectories. More precisely, the robots share information regarding noisy trajectories that are determined without odometry error corrections (true positions may significantly differ from those that are shared).

If robots cannot share information on a regular basis (e.g. if robots are far away from each other), the standardized projected probability $P_t^M(\overrightarrow{r_0}|\Theta_t)$ is updated as a function of the damped mass center of detections made by all the robots and of locations of the robot having index k where no detection has been made if such information is available (i.e. when robots can exchange information) or as a function of the damped mass center of detections made by the robot and of its locations where no detection has been made (i.e. when robots cannot exchange information).

To determine its next position, each robot minimizes the free energy $F_t(\Theta_t)$ as described above. As mentioned above, each robot takes individual decisions to generate its own path and information is shared through the part of the path that each robot shares.

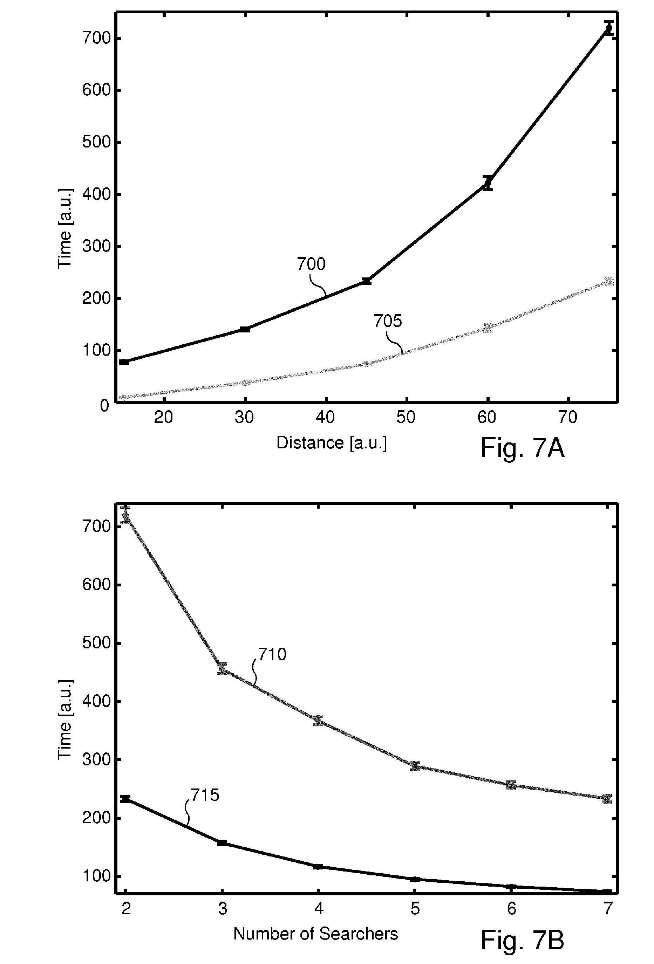

**Figure 7,** comprising Figures 7a and 7b, illustrates an advantage of using several robots to locate one or several emitting sources.

Figure 7a illustrates the variation of the average searching time as a function of the average distance between the robots and the centers of two emitting sources (which are distant from each other by distance $\lambda$ = 30 a.u.) and as a function of the number of robots used. Curve 700 represents the variation of the average searching time when two robots are used and curve 705 represents the variation of the average searching time when seven robots are used.

Figure 7b illustrates the variation of the average searching time as a function of the number of robots and as a function of the distance between the robots and the centers of two emitting sources (which are distant from each other by distance $\lambda$ = 30 a.u.). Curve 710 represents the variation of the average searching time when the distance between the robots and the centers of two emitting sources is equal to 75 a.u. and curve 715 represents the variation of the average searching time when the distance between the robots and the centers of two emitting sources is equal to 45 a.u..

Although the present invention has been described hereinabove with reference to specific embodiments, the present invention is not limited to the specific embodiments, and modifications will be apparent to a skilled person in the art which lie within the scope of the present invention.

Many further modifications and variations will suggest themselves to those versed in the art upon making reference to the foregoing illustrative embodiments, which are given by way of example only and which are not intended to limit the scope of the invention, that being determined solely by the appended claims. In particular the different features from different embodiments may be interchanged, where appropriate.

In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used.

**Claims**

1. A mobile device for real-time monitoring of odor detection by a moving animal or by a moving apparatus, in turbulent flows, the device comprising the following means:

   - connecting means (200) for connecting to the device at least a portion of biological material that is sensitive to at least one predetermined molecule, the at least a portion of biological material producing a specific electrical signal in the presence of the at least one predetermined molecule;
   - amplification means (205, 220) for amplifying an electrical signal received from the connecting means and providing an amplified signal to computation means; and
   - computation means (250) for filtering an amplified signal received from the amplification means, the filtering comprising a step of applying a temporal high-pass filter to the amplified signal received from the amplification means.

**2.** The device according to claim 1 wherein the computation means are further configured for generating a signal representing the detection or absence of detection of the at least one predetermined molecule, as a function of the filtered amplified signal.

**3.** The device according to claim 1 or claim 2 wherein the amplification means comprise at least one of an instrument amplifier (205) and an operational amplifier (220).

**4.** The device according to any one of claims 1 to 3 further comprising wireless emitting means for emitting a signal obtained in the computation means as a function of the amplified signal received from the amplification means.

**5.** The device according to any one of claims 1 to 4 further comprising powering means, the powering means comprising a power circuit (260) for providing a virtual ground.

**6.** The device according to any one of claims 1 to 5 wherein the electrical signal received from the connecting means is of the ElectroAntennoGram type.

**7.** The device according to any one of claims 1 to 6 further comprising a circuitry for modelling the at least a portion of biological material as a source of voltage.

**8.** The device according to any one of claims 1 to 7 further comprising the at least a portion of biological material, the at least a portion of biological material including at least a portion of an insect antenna.

**9.** The device according to any one of claims 1 to 8 wherein the connecting means are configured for connecting a plurality of portions of biological material to the device, each of the portions of biological material being sensitive to at least one predetermined molecule and producing a specific electrical signal in the presence of the predetermined molecule, each of the portions of biological material providing a specific temporal dynamic and a specific sensibility in the presence of the predetermined molecule to which the portion of biological material is sensitive.

**10.** The device according to any one of claims 1 to 9, depending on claim 2, further comprising actuator controller means for controlling displacements of an apparatus comprising the device, the actuator controller means providing a control signal that is at least partially determined as a function of the signal representing the detection or absence of detection of the at least one predetermined molecule.

**11.** The device according to any one of claims 1 to 10 wherein the computation means are further configured for:

- computing, for a plurality of possible next locations of the device, a free energy variation for moving the device from its current location to each of the plurality of possible next locations, the free energy being computed as a function of a standardized projected probability field of the location of a diffusing source of the at least one predetermined molecule, based on previous emission propagation measurements of the at least one predetermined molecule, the previous emission propagation measurements being obtained along a self-generated path of the device,
- determining a minimum free energy variation value amongst the computed free energy variations; and
- identifying the location associated with the determined minimum free energy variation as being the next location of the device.

**12.** A method to be carried out in a mobile device for real-time monitoring of odor detection by a moving animal or by a moving apparatus, in turbulent flows, the method comprising the steps of:

- receiving an electrical signal from at least a portion of biological material that is sensitive to at least one predetermined molecule, the at least a portion of biological material producing a specific electrical signal in the presence of the at least one predetermined molecule;
- amplifying the received electrical signal; and
- filtering the amplified signal, the filtering step comprising a step of applying a temporal high-pass filter to the amplified received signal.

**13.** The method according to claim 12 further comprising a step of wirelessly emitting the filtered signal.

**14.** The method according to claim 12 further comprising a step of generating a signal representing the detection or absence of detection of the at least one predetermined molecule as a function of the filtered amplified signal.

15. The method according to claim 14 further comprising a step of wirelessly emitting the signal representing the detection or absence of detection of the at least one predetermined molecule.

16. The method according to claim 14 or claim 15 further comprising a step of controlling a displacement actuator at least partially as a function of the signal representing the detection or absence of detection of the at least one predetermined molecule.

17. The method according to any one of claims 12 to 16 further comprising the steps of:

- computing, for a plurality of possible next locations of the device, a free energy variation for moving the device from its current location to each of the plurality of possible next locations, the free energy being computed as a function of a standardized projected probability field of the location of a diffusing source of the at least one predetermined molecule, based on previous emission propagation measurements of the at least one predetermined molecule,
- determining a minimum free energy variation value amongst the computed free energy variations; and
- identifying the location associated with the determined minimum free energy variation as being the next location of the device.

18. The method according to any one of claims 12 to 17 wherein the electrical signal received from the at least a portion of biological material is of the ElectroAntennoGram type.

19. The method according to any one of claims 12 to 18 wherein the at least one predetermined molecule is mixed with particles, molecules, or fragments of molecules that a source is to be located.

Fig. 1

(a)

(b)

Fig. 2

Fig. 3

Fig. 4

(a)

(b)

(c)

(d)

530 — user interface

535 — communication interface

540 — mass storage

545 — sensor

550 — actuator controller

555

520

I/O

525

500

CPU — 505

ROM — 510

RAM — 515

## Fig. 5

600 — Determining a mapless search function $F_t(\Theta_t)$

605 — Obtaining measurement of propagation at sensor location ($\vec{r}_t$)

610 — Source located? —yes→ end

no

615 — Determining a set of $\vec{r}^i_{t+dt}$

620 — Determining $\vec{r}_{t+dt}$ minimizing $F_t (\vec{r}_t \rightarrow \vec{r}_{t+dt}|? _t)$ for each $\vec{r}^i_{t+dt}$ of the set

625 — Moving sensor to $\vec{r}_{t+dt}$

610' — Source located? —yes→ end

no

## Fig. 6

Fig. 7A

Fig. 7B

**EUROPEAN SEARCH REPORT**

Application Number

EP 14 30 6246

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DOMINIQUE MARTINEZ ET AL: "Using Insect Electroantennogram Sensors on Autonomous Robots for Olfactory Searches", JOURNAL OF VISUALIZED EXPERIMENTS, vol. 90, 4 August 2014 (2014-08-04), pages 1-9, XP055150433, DOI: 10.3791/51704 | 1-8,10, 12-16, 18,19 | INV. G01N33/00 |
| Y | * abstract * <br> * Sections 1-4; page 2 * <br> * last paragraph; page 3 * <br> * figures 1,2,6 * | 9,11,17 | |
| Y | MYRICK A J ET AL: "Real-time odor discrimination using a bioelectronic sensor array based on the insect electroantennogram; Real-time odor discrimination using a bioelectronic sensor array", BIOINSPIRATION & BIOMIMETICS, INSTITUTE OF PHYSICS PUBLISHING, BRISTOL, GB, vol. 3, no. 4, 046006, 1 December 2008 (2008-12-01), pages 1-19, XP020148359, ISSN: 1748-3190, DOI: 10.1088/1748-3182/3/4/046006 <br> * abstract * <br> * figures 2,3 * | 9 | |

TECHNICAL FIELDS SEARCHED (IPC)

G01N

-/--

The present search report has been drawn up for all claims

| Place of search | Date of completion of the search | Examiner |
|---|---|---|
| The Hague | 6 November 2014 | Couteau, Olivier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 14 30 6246

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | J.-B. MASSON: "Olfactory searches with limited space perception", PROCEEDINGS OF THE NATIONAL ACADEMY OF SCIENCES, vol. 110, no. 28, 9 July 2013 (2013-07-09), pages 11261-11266, XP055151177, ISSN: 0027-8424, DOI: 10.1073/pnas.1221091110 * abstract * * page 11262, left-hand column, paragraph 1 - right-hand column, paragraph 2 * * page 11264, right-hand column, paragraph 3 * * page 11265, right-hand column, paragraph 3 * | 11,17 | |

-----

TECHNICAL FIELDS
SEARCHED        (IPC)

The present search report has been drawn up for all claims

| Place of search | Date of completion of the search | Examiner |
|---|---|---|
| The Hague | 6 November 2014 | Couteau, Olivier |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 ...........................................................................

& : member of the same patent family, corresponding
    document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009271125 A

- EP 1881389 A